# EUROPEAN PATENT APPLICATION

(11) **EP 4 371 581 A1**
(43) Date of publication of application: **22.05.2024**
(21) Application number: 23207080.5
(22) Date of filing: 31.10.2023
(51) Int. Cl.: A61L 2/06, A61L 2/14, A61L 2/08

(54) **DRYING CABINET FOR REUSABLE INVASIVE MEDICAL DEVICES**

(30) Priority: 03.11.2022 IT 202200022512
(71) Applicant: MDG Engineering S.r.l., 48124 Ravenna (IT)
(72) Inventor: ZANIBONI, Benedetta, RAVENNA (IT)
(74) Representative: Dall'Olio, Christian

(57) **Abstract**

The drying cabinet (100) of invasive medical devices which are to be reutilised after a washing process comprises at least a drying chamber (1), for receiving and storing, internally thereof, one or more medical devices to be dried after a washing process; an air circulation circuit, for circulation of an air flow across the drying chamber.

The drying cabinet (100) comprises, along the air circulation circuit, an air purification filter (4), an air heating device (6), downstream of the air purification filter (4), and a disinfection module (7).

The disinfection module (8) is arranged downstream of the air heating device (6) so as to be crossed by the air flow, in such a way that a disinfecting air flow is injected into the drying chamber (1).

## Description

The present invention concerns the technical sector relating to the drying of invasive medical devices destined, after use thereof, to be reutilised following a proper washing process and a subsequent drying.

Reutilisable invasive medical devices can be constituted by any instrument or apparatus, destined to be used in the human sphere with the aim of: diagnosis, checking or surgery using total or partial penetration into the body via an orifice or a body surface, and which can newly be reutilised following appropriate washing and drying.

In fact, following use of an invasive medical device, in order to be reutilised, the device must firstly be washed, with water or other washing liquids and detergents.

Since residual moisture (water or washing liquids) on the medical device at the end of the washing process can facilitate a bacterial proliferation, the device must be completely dried in order then to be subjected to a disinfection in preparation for its further use.

This is of particular importance for a series of reutilisable invasive medical devices which comprise a series of internal channels which must be guaranteed to be aseptic at moment of use.

Among these can be mentioned, for example, diagnostic and control probes such as the gastroscope, colonoscope, duodenoscope, echoendoscope, cystoscope, bronchoscope, enteroscope.

To carry out the drying of these medical devices on termination of the washing process thereof, use is made of drying cabinets.

Usually, a drying cabinet comprises at least a drying chamber which is configured so as to be able to receive, and store internally thereof, the medical devices to be subjected to drying.

The drying cabinet also comprises an air circulation circuit and at least a conveying device of an air flow along the air circulation circuit.

The conveying device (for example a ventilator device) is arranged in a technical housing present in the drying cabinet, separated by a relative dividing wall from the at least a drying chamber.

The air circulation circuit comprises air passage conduits which are in communication, on one side, with the conveying device, present inside the technical housing, and, on the other side, with the inside of the at least a drying chamber.

The dividing wall between the drying chamber and the technical housing offers appropriate air passages for establishing communication of the drying chamber with the technical housing.

In the technical housing, arranged downstream of the conveying device of the air flow, there is an air purification filter, with a capacity for bacterial separation from the air flow crossing it.

The conveying device of an air flow is thus able to convey an air flow across the air purification filter and then along the air circulation circuit so that the purified air flow from the filter enters the drying chamber, crosses the drying chamber in which the medical devices to be dried are placed, and returns into the technical housing through the holes present in the dividing wall.

To accelerate the drying process, and to completely abate the residual moisture present on the medical devices situated inside the drying chamber, the drying cabinets are provided with heating devices (for example batteries of electrical resistors) which are positioned along the air circulation circuit, downstream of the air purification filter and upstream of the drying chamber, in such a way as to be able to heat the air before it is injected into the drying chamber.

The drying cabinets are without doubt effective in drying the medical devices and in abating the level of residual moisture therein, thus preventing any bacterial proliferation.

However, they are less effective in wholly eliminating any bacterial loads that might be residual in the medical devices at the end of the previous washing process.

Further, drying cabinets made in this way are not at all effective in eliminating any external contaminations, either of the bacterial type, or, and especially, of the viral type, to which the medical devices may be exposed during the passage thereof, or transfer thereof, from the washing device to inside the drying chamber.

Further, the drying chamber itself can be subject to external contaminations, during the steps of inserting and/or extracting of the medical devices, which cannot be eliminated or abated merely by circulation of a heated and purified air flow.

The aim of the present invention is therefore to provide a new drying cabinet that is able to obviate the above-mentioned drawbacks.

In particular, an aim of the present invention is to provide a new drying cabinet able to carry out disinfection of the medical devices arranged in the drying chamber as well as disinfection of the drying chamber itself, both during a normal drying cycle, and before or after the storage of the medical devices inside the drying chamber.

The characteristics of the invention will be described in the following in which some preferred but not exclusive embodiments of the drying cabinet will be described with reference to the accompanying tables of drawings, in which:

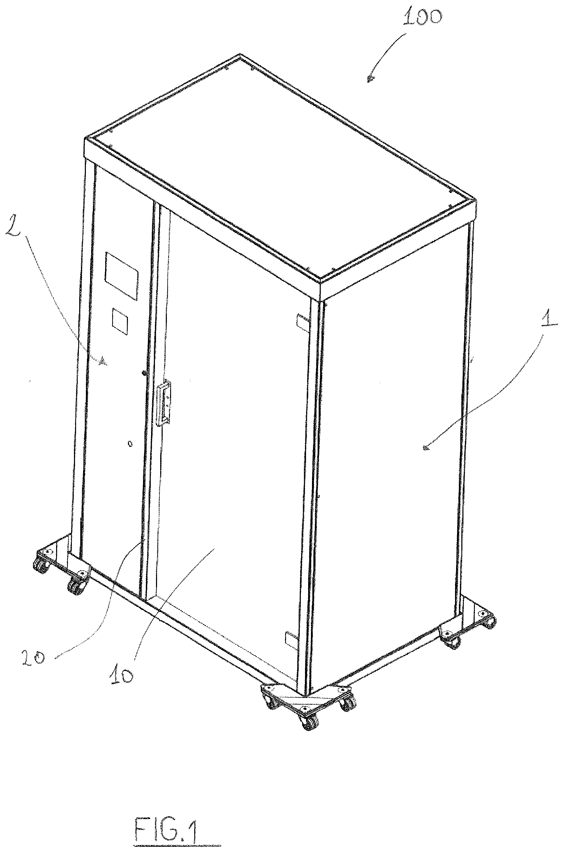

- figure 1 is a schematic perspective view of the outside of the drying cabinet of the present invention;
- figure 2 is a schematic perspective view of the inside of the drying cabinet of the invention according to a first possible embodiment;
- figure 3 is a frontal view, with some parts removed, of the drying cabinet illustrated in figure 2;
- figure 4 is a frontal view, with some parts removed, of the drying cabinet illustrated of the invention in a possible further embodiment of the invention;
- figures 5 and 6 illustrate, in respective perspective views sectioned according to different section planes, the drying cabinet illustrated in figure 4.

With reference to the appended tables of drawings, reference numeral (100) denotes the drying cabinet of invasive medical devices which are to be reutilised after a washing process, object of the present invention in its entirety.

In all the illustrated embodiments, the drying cabinet (100) comprises the following characteristics.

It comprises:
at least a drying chamber (1), which is conformed and configured to receive and
store, internally thereof, one or more medical devices to be dried after a washing process;
a door (10), for opening and closing the drying chamber (1);
a service chamber (2), separated by a dividing wall (20) from the at least a drying chamber (1).

The drying cabinet (100) further comprises:
a conveying device (3) of an air flow, which is arranged in the service chamber (2); an air circulation circuit (5), for circulation of an air flow, comprising at least an air passage conduit (51) communicating, on one side, with the service chamber (2) and the conveying device (3) of an air flow and, on the other side with the inside of the at least a drying chamber (1) and comprising air passage holes at the dividing wall (20), for the passage of air from the at least a drying chamber (1) to the service chamber (2);
an air purification filter (4), also arranged in the service chamber (2) and having an inlet (41) communicating with the conveying device (3) of an air flow and having an outlet (42) communicating with the at least an air passage conduit (51).

The conveying device (3) of an air flow, the air purification filter (4) and the air circulation circuit (5) are reciprocally arranged in such a way that the conveying device (3) of an air flow generates and directs an air flow (schematically represented with arrows in the figures which indicate the pathway thereof) to cross the air purification filter (4) and then subsequently to cross the at least an air passage conduit (51) in order to be injected internally of the at least a drying chamber (1) and to cross the drying chamber (1) then to return to the service chamber (2) via the passage holes of the dividing wall (20).

For the purpose of drying and abating the residual moisture present in the medical devices which are inserted and stored inside the at least a drying chamber (1), the drying cabinet (100) also comprises an air heating device (6) which is arranged along the at least an air passage conduit (51), downstream of the air purification filter (4) and upstream of the at least a drying chamber (1), and which is configured, when activated, to heat the purified air flow before it reaches internally of the at least a drying chamber (1).

The heating device (6), for example, can comprise batteries of electrical resistors that are activatable and regulatable by means of control of relative temperature detecting sensors.

The special characteristics of the drying cabinet (100) object of the present invention consist in the fact that it comprises a disinfection module (7) which is arranged along the at least an air passage conduit (51), in a position downstream of the air heating device (6) and upstream of the drying chamber (1), in such a way as to be crossable by the purified air flow.

The disinfection module (7) comprises a plasma disinfecting device (71) activatable for the disinfection of the purified air flow before the air flow reaches internally of a drying chamber (1) in such a way that a disinfecting air flow is injected into the at least a drying chamber (1).

In greater detail, the plasma disinfecting device (71) is activatable during both the drying of medical devices present inside the at least a drying chamber (1), in such a way that the medical devices, as well as being subjected to drying, are also subjected to a total disinfection, and when the at least a drying chamber (1) is empty, between a drying cycle already carried out and a subsequent drying cycle still to be carried out, so that the at least a drying chamber (1) is subjected to a total disinfection before receiving other medical devices to be dried.

Owing to the presence of the disinfection module (7), comprising the plasma disinfecting device (71), it is thus possible to carry out a disinfection of the drying chamber as well as of the medical devices inserted and stored internally thereof, to be dried after the washing process.

In this way, the medical devices, as well as being completely dried, are also disinfected so as to completely eliminate any residues of contaminations present thereof whether bacterial or viral.

The eventual contaminations on the medical devices which are inserted and stored inside the at least a drying chamber for drying thereof might still be present at the end of the preceding washing process, or be deposited on the medical devices during the transfer operations from the washing device inside the at least a drying chamber of the drying cabinet.

With the disinfection module, the drying cabinet is also able to guarantee the disinfection of the at least a drying chamber even when it is not directly used for drying the medical devices, for example the drying chamber can be subjected to disinfection between one drying process and the other, or after each opening.

In fact, the opening of the door of the drying chamber can be the cause of eventual contaminations originating from the outside environment, determined by the type of air circulation present in the outside environment.

Therefore, and definitively, the drying cabinet of the present invention enables obviating the drawbacks present in the drying cabinets of the prior art, as previously defined, guaranteeing not only the carrying out of the drying of the medical devices introduced therein, but also the disinfection thereof, as well as the disinfection of the at least a drying chamber in which the medical devices are stored.

Further and other advantageous characteristics of the drying cabinet are described in the following.

The drying cabinet (100) can be realised in such a way that the disinfection module (7) further comprises also a photocatalytic device (72) and an emitting element (73) of a light radiation for activation of the photocatalytic device (72) in such a way as to generate a photocatalysis for a photocatalytic disinfection of the air flow before it reaches inside the at least a drying chamber (1).

In particular, the emitting element (73) of a light radiation is activatable to activate the photocatalytic device (72) during both the drying process of medical devices present inside the at least a drying chamber (1), in such a way that the medical devices, as well as being subjected to drying, are also subjected to a further disinfection by photocatalysis, and when the at least a drying chamber (1) is empty, between a drying cycle already carried out and a subsequent drying cycle still to be carried out, in such a way that the at least a drying chamber (1) is subjected to a further disinfection by photocatalysis before receiving other medical devices to be dried.

The presence of a plasma disinfecting device and a disinfection by photocatalysis device guarantees a complete and optimal disinfection of both the medical devices, during the drying thereof, and of the drying chamber.

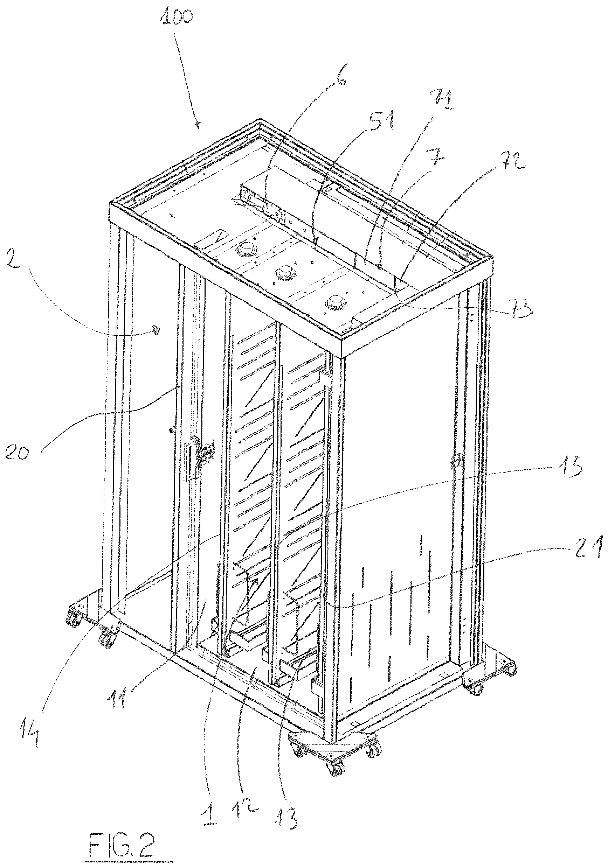

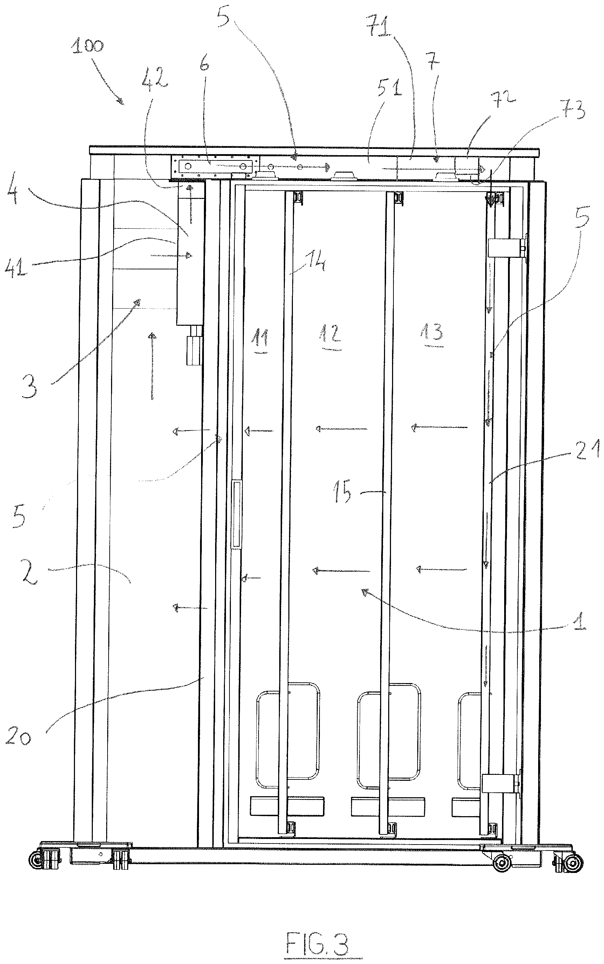

Further, in a first preferred embodiment, such as for example illustrated in figures 2 and 3, the drying cabinet (100) can be realised in such a way that the at least a drying chamber (1) comprises a plurality of compartments (11, 12, 13), each of which is conformed and configured for insertion and storing of respective medical devices, separated from one another by a respective plurality of bulkheads (14, 15) comprising, internally thereof, gaps for the passage of air and having air passage holes to enable passage of air between the compartments (11, 12, 13) For example, in particular, the plurality of compartments (11, 12, 13) can comprise a first external compartment (11), bordering the service chamber (2) and separated from the service chamber (2) by the dividing wall (20), and a second external compartment (13), delimited by a second dividing wall (21) communicating with the at least an air passage conduit (51).

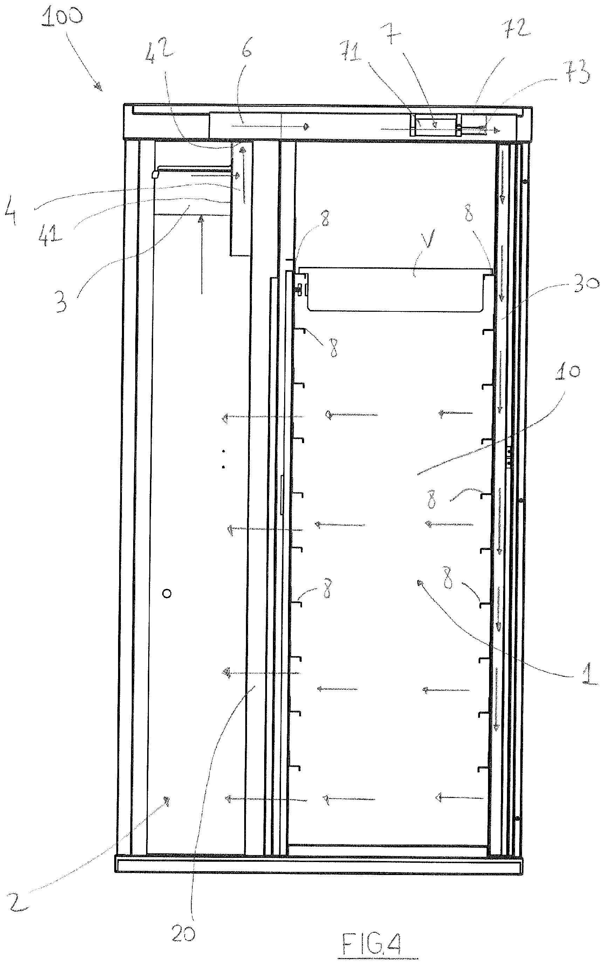

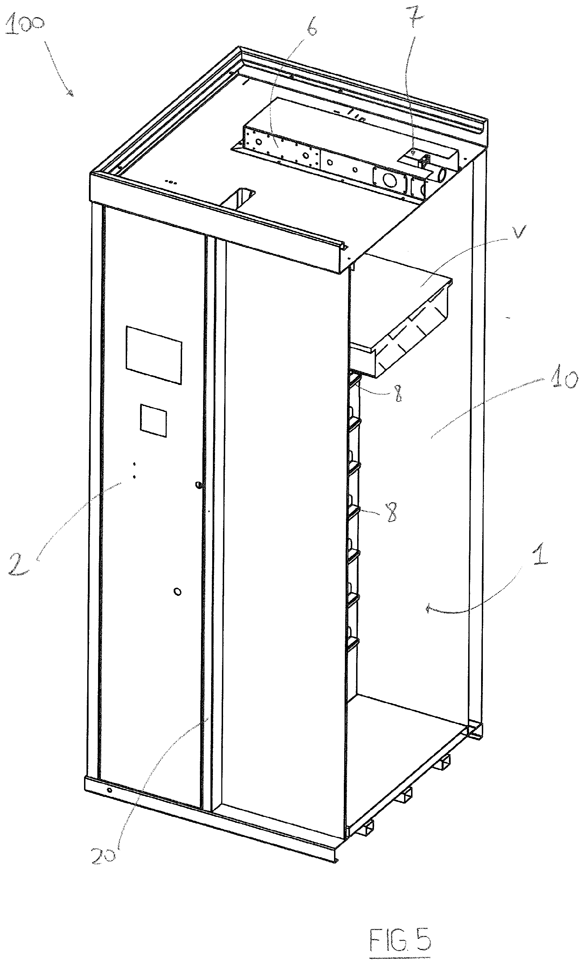

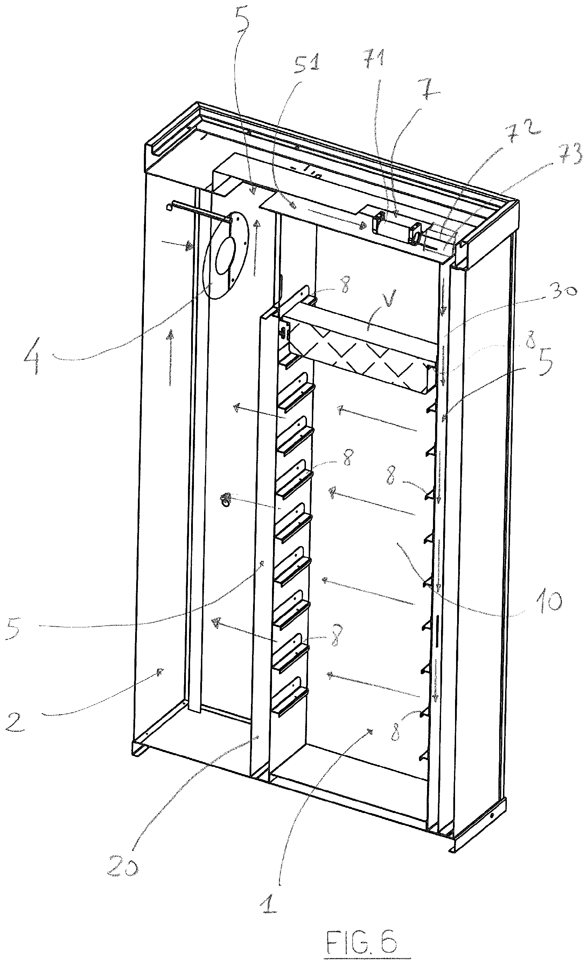

Alternatively, in a further and other possible embodiment, illustrated in figures 4, 5 and 6, the drying cabinet (100) can be realised in such a way that the at least a drying chamber (1) comprises a single compartment (10) which is configured and conformed for insertion and storing of respective medical devices.

The single compartment (10) is thus bordering the service chamber (2) and separated from the service chamber (2) by the dividing wall (20) and also comprises a second dividing wall (30) which is in communication, for example by means of air passage holes and a relative gap internal thereof, with the at least an air passage conduit (51).

The single compartment (10) can comprise, at the dividing wall (20) with the service chamber (2) and at the second dividing wall (30), a plurality of support elements (8) opposite one another, and arranged located vertically away from one another, which are configured for providing support to containing trays (V) of medical devices.

## Claims

1. A drying cabinet (100) of invasive medical devices which are to be re-utilised after a washing process, comprising:
at least a drying chamber (1), conformed and configured to receive, and store internally thereof, one or more medical devices to be dried after a washing process;
a door (10), for opening and closing the drying chamber (1);
a service chamber (2), separated by a dividing wall (20) from the at least a drying chamber (1);
a conveying device (3) of an air flow, arranged in the service chamber (2);
an air circulation circuit (5), for circulation of an air flow, comprising at least an air passage conduit (51) communicating, on one side, with the service chamber (2) and with the conveying device (3) of an air flow and, on the other side, with the inside of the at least a drying chamber (1) and comprising air passage holes at the dividing wall (20), for the passage of air from the at least a drying chamber (1) to the service chamber (2);
an air purification filter (4), having an inlet (41) communicating with the conveying device (3) of an air flow and having an outlet (42) communicating with the at least an air passage conduit (51),
wherein the conveying device (3) of an air flow, the air purification filter (4) and the air circulation circuit (5) are reciprocally arranged in such a way that the conveying device (3) of an air flow generates and directs an air flow to cross the air purification filter (4) and then subsequently to cross the at least an air passage conduit (51) in order to be injected internally of the at least a drying chamber (1) and to cross the drying chamber (1) then to return to the service chamber (2) via the passage holes of the dividing wall;
an air heating device (6), arranged along the at least an air passage conduit (51), downstream of the air purification filter (4) and upstream of the at least a drying chamber (1), and configured, when activated, to heat the purified air flow before it reaches internally of the at least a drying chamber (1),
**characterised in that** it comprises a disinfection module (7) arranged along the at least an air passage conduit (51), in a position downstream of the air heating device (6), and upstream of the drying chamber (1), in such a way as to be crossable by the purified air flow, wherein the disinfection module (7) comprises a plasma disinfecting device (71) activatable for the disinfection of the purified air flow before the air flow reaches the inside of the drying chamber (1) in such a way that a disinfecting air flow is injected into the at least a drying chamber (1), wherein the plasma disinfecting device (71) is activatable during both the drying of medical devices present internally of the at least a drying chamber (1), in such a way that the medical devices, as well as being subjected to drying are also subjected to a total disinfection, and when the at least a drying chamber (1) is empty, between a drying cycle already carried out and a subsequent drying cycle still to be carried out, in such a way that the at least a drying chamber (1) is subjected to a total disinfection before receiving other medical devices to be dried.

2. The drying cabinet (100) as claimed in claim 1, wherein the disinfection module (7) further comprises a photocatalytic device (72) and an emitting element (73) of a light radiation for activation of the photocatalytic device (72) in such a way as to generate a photocatalysis for a photocatalytic disinfection of the air flow before it reaches the inside of the at least a drying chamber (1), wherein the emitting element (73) of a light radiation is activatable to activate the photocatalytic device (72) during both the drying of medical devices present internally of the at least a drying chamber (1), in such a way that the medical devices, as well as being subjected to drying are also subjected to a disinfection by photocatalysis, and when the at least a drying chamber (1) is empty, between a drying cycle already carried out and a subsequent drying cycle still to be carried out, in such a way that the at least a drying chamber (1) is also subjected to a disinfection by photocatalysis before receiving other medical devices to be dried.

3. The drying cabinet (100) as claimed in any one of the preceding claims, wherein the at least a drying chamber (1) is realised in such a way as to comprise a plurality of compartments (11, 12, 13), each of which is conformed and configured for insertion and storing of respective medical devices, and separated from one another by a respective plurality of bulkheads (14, 15) comprising, internally thereof, gaps for the passage of air and having air passage holes to enable passage of air between the compartments (11, 12, 13), wherein the plurality of compartments (11, 12, 13) comprises a first external compartment (11), bordering the service chamber (2) and separated from the service chamber (2) by said dividing wall (20), and a second external compartment (13), delimited by a second dividing wall (21) communicating with the at least an air passage conduit (51).

4. The drying cabinet (100) as claimed in any one of claims from 1 to 2, wherein the at least a drying chamber (1) is realised in such a way as to comprise a single compartment (10) configured and conformed for insertion and storing of respective medical devices, wherein the only compartment (10) borders with the service chamber (2) and separated from the service chamber (2) by said dividing wall (20) and comprises a second dividing wall (30) communicating with the at least an air passage conduit (51).

5. The drying cabinet (100) as claimed in claim 4, wherein the single compartment (10) comprises, at the dividing wall (20) with the service chamber (2) and at the second dividing wall (30), a plurality of support elements (8) opposite one another, and arranged located vertically away from one another, for providing support to containing trays (V) of medical devices.
